# EUROPÄISCHE PATENTANMELDUNG

(11) **EP 1 209 222 A2**
(43) Veröffentlichungstag der Anmeldung: **29.05.2002**
(21) Anmeldenummer: 01126882.8
(22) Anmeldetag: 12.11.2001
(51) Int. Cl.: C12M 1/00

(54) **Objektlagerstation und Klimaschrank**

(30) Priorität: 24.11.2000 DE 10058564
(71) Anmelder: KENDRO Laboratory Products GmbH, 63450 Hanau (DE)
(72) Erfinder: Ferger, Stefan, 63691 Ranstadt (DE); Heeg, Hubert, 63776 Mömbris (DE); Jelinski, Sonja, 63594 Hasselroth (DE)
(74) Vertreter: Lang, Friedrich, Dipl.-Ing.

(57) **Zusammenfassung**

Die Erfindung betrifft eine Objektlagerstation mit einer Vorderfront zur Beschickung mit Objekten, einer Rückseite und zwei gegenüberliegend angeordneten Seitenwänden und mit an den Seitenwänden jeweils paarweise gegenüberliegend im Inneren der Objektlagerstation im Wesentlichen horizontal erstreckend angeordneten Objekt-Aufnahmeschienen mit Auflageflächen und besteht darin, dass die Seitenwände mehrere gleiche, sich waagerecht erstreckende, übereinander angeordnete Durchbrüche aufweisen, die in beiden Seitenwänden in gleicher Anzahl und Lage und is stets gleichem Abstand von benachbarten Durchbrüchen angeordnet sind, wobei die Anzahl der Durchbrüche jeder Seitenwand der Anzahl der Objekt-Aufnahmeschienen der Seitenwand entspricht und dass jeder Durchbruch jeweils den gleichen Abstand zu der jeweils nächstliegenden Objekt-Aufnahmeschiene aufweist. Des Weiteren betrifft die Erfindung eine lösbare Befestigung der Aufnahmeschienen.

## Beschreibung

Die Erfindung betrifft eine Objektlagerstation mit einer Vorderfront zur Beschickung von Objekten, einer Rückseite und zwei gegenüberliegend angeordneten Seitenwänden und mit an den Seitenwänden jeweils paarweise gegenüberliegend im Inneren der Objektlagerstation im Wesentlichen horizontal erstreckend angeordneten Objekt-Aufnahmeschienen mit Auflageflächen. Des Weiteren betrifft die Erfindung einen Klimaschrank.

Derartige Objektlagerstationen sind aus WO 98/05753 bekannt. Die hier offenbarten Objektlagerstationen weisen an ihren Seiten Öffnungen auf. Diese Öffnungen sind in unterschiedlicher Weise zu den in von den Objektlagerstationen aufzunehmenden Objekten, nämlich zu den Mikrotiterplatten, angeordnet. Dadurch ergeben sich bei den einzelnen Mikrotiterplatten unterschiedliche durch die Öffnungen geführte Luftströmungen. Derart unterschiedliche Luftströmungen führen zu unterschiedlichen Wachstumsbedingungen der in den Mikrotiterplatten vorgesehenen Präparate. Die unterschiedliche Anordnung der Mikrotiterplatten in Bezug auf die seitlichen Öffnungen führt zu ungleichmäßigen Verunreinigungen der Objektlagerstation, was die Autoklavierbarkeit komplizierter gestaltet und deren Erfolg herabsetzt. Die Objekte werden auf aus den Seitenteilen ausgestanzten Aufnahmen geführt. Diese Aufnahmen sind punktförmig angelegt. Mit ihnen ist eine Auflage der Mikrotiterplatten lediglich im vorderen und im hinteren Bereich gegeben, so dass das Einschieben der Mikrotiterplatten in die Objektlagerstation exakt waagerecht erfolgen muss, um ein Verklemmen der Mikrotiterplatten zu vermeiden. Die ausgestanzten Halterungen sind konstruktionsbedingt relativ schwer zu autoklavieren.

Aufgabe der vorliegenden Erfindung ist es, die bekannten Lösungen zu verbessern und insbesondere die Autoklavierbarkeit von Objektlagerstationen zu verbessern.

Die Aufgabe wird erfindungsgemäß durch die kennzeichnenden Merkmale der unabhängigen Ansprüche gelöst. Dadurch, dass die Seitenwände erfindungsgemäß mehrere gleiche, sich waagerecht erstreckende, übereinander angeordnete Durchbrüche aufweisen, die in beiden Seitenwänden in gleicher Anzahl und Lage und stets in gleichem Abstand von benachbarten Durchbrüchen angeordnet sind, wobei die Anzahl der Durchbrüche jeder Seitenwand der Anzahl der Objekt-Aufnahmeschienen der Seitenwand entspricht und dass jeder Durchbruch jeweils den gleichen Abstand zu der jeweils nächstliegenden Objekt-Aufnahmeschiene aufweist, ist eine leichte Autoklavierbarkeit gegeben, da die gesamte Fläche relativ gleichmäßig verunreinigt ist. Des Weiteren hat eine derartige Anordnung den Vorteil, dass die aufzunehmenden Objekte, beispielsweise Mikrotiterplatten, von einer seitlichen Luftströmung gleichmäßig umströmt werden, so dass sich gleichmäßige Wachstumsbedingungen in allen übereinander angeordneten Mikrotiterplatten ausbilden können. Damit kann das Zellwachstum innerhalb der Mikrotiterplatten optimiert werden.

Insbesondere ist es vorteilhaft, dass die Objekt-Aufnahmeschienen unterhalb der Durchbrüche angeordnet sind. Dadurch sind die Seiten der Objekte (beispielsweise Mikrotiterplatten) geschützt und der Luftstrom bewegt sich oberhalb der Objekte. Insbesondere ist es für eine einfachere Wartung vorteilhaft, dass die Objekt-Aufnahmeschienen lösbar an den Seitenteilen angeordnet sind. Dies ist insbesondere von Vorteil, wenn die Objekt-Aufnahmeschienen aus Kunststoff gebildet sind. Sie können jedoch auch aus Metall, beispielsweise aus Edelstahl (wie eventuell die übrigen Teile der Objektlagerstation), und fest an den Seitenwänden angeordnet sein. Von Vorteil ist es weiterhin, dass die Objekt-Aufnahmeschienen zumindest an ihrem der Vorderfront zugewandten Ende auf ihrer Oberseite jeweils mindestens ein Anschlagelement aufweisen. Dadurch wird verhindert, dass sich die Objekte selbständig aus der Lagerstation herausbewegen, wenn diese, beispielsweise beim Transport, eventuell geneigt wird. Zweckmäßig ist es, dass an den Ober- und Unterseiten der Objekt-Aufnahmeschienen an deren der Vorderfront und/oder der Rückseite zugewandten Enden jeweils gleiche Anschlagelemente angeordnet sind. Dadurch sind standardisierte Objekt-Aufnahmeschienen an beiden Seitenwänden der Objektlagerstation in gleicher Weise einsetzbar.

Insbesondere ist es vorteilhaft, dass an den den Seitenwänden zugewandten Kanten der Objekt-Aufnahmeschienen Stützleisten angeordnet sind, die an der jeweiligen Seitenwand anliegen und sich mindestens oberhalb der Auflageflächen der Objekt-Aufnahmeschienen erstrecken, wobei der senkrecht zur Seitenwand und senkrecht zur Auflagefläche der Objekt-Aufnahmeschiene gebildete Querschnitt der Stützleisten an der Verbindungsstelle zur Objekt-Aufnahmeschiene eine größere, senkrecht zur Seitenwand gemessene Breite aufweist, als der des der Verbindungsstelle abgewandten Endes. In diesem Zusammenhang ist es zweckmäßig, dass die Breite des Querschnitts stetig geändert ist. Dadurch wird eine schiefe Ebene auf beiden Seiten der Objekt-Aufnahmeschiene gebildet, so dass die aufzunehmenden Objekte beim Einsetzen zentriert werden. Toleranzen in der Breite oder Positionierung der Objekte können dadurch ausgeglichen werden. Die Stützleiten können auch unterhalb der Objekt-Aufnahmeschienen erstreckt sein und dadurch zu einer Erhöhung der Stabilität der Objekt-Aufnahmeschienen beitragen.

Eine zweite Ausführungsform der Erfindung ist dadurch gekennzeichnet, dass die Objekt-Aufnahmeschienen jeweils in mindestens zwei in den Seitenwänden horizontal nebeneinander angeordneten Befestigungsösen mittels an den Aufnahmeschienen angeordneter Befestigungsnippel gehaltert sind, wobei die Befestigungsösen sich in vertikaler Richtung erstrecken und wobei ein Befestigungsnippel jeweils einer Objekt-Aufnahmeschiene im unteren Bereich der zugeordneten Befestigungsöse arretiert ist und der mindestens eine weitere Befestigungsnippel im unteren Bereich der zugeordneten Befestigungsöse horizontal-verschiebbar gehalten ist. Auf diese Weise sind die Objekt-Aufnahmeschienen zwar definiert aber trotzdem lösbar gehalten. Durch Arretierung eines Befestigungsnippels bei gleichzeitiger Verschiebbarkeit weiterer Befestigungsnippel wird ein unterschiedliches temperaturabhängiges Ausdehnungsverhalten der Seitenwände der Objektlagerstation einerseits und der Objekt-Aufnahmeschienen andererseits ausgeglichen. Dies ist insbesondere von Bedeutung, wenn die Objekt-Aufnahmeschienen aus einem Kunststoff gebildet sind, während die Seitenwände der Objektlagerstation üblicherweise aus Metall bestehen.

Zweckmäßig ist es, dass die Objekt-Aufnahmeschienen jeweils mit drei Befestigungsnippeln an einer Seitenwand gehaltert sind, wobei es insbesondere vorteilhaft ist, dass der mittlere Befestigungsnippel in der zugeordneten Befestigungsöse arretiert ist. Dies sichert eine gleichmäßige Ausdehnung an beiden Enden der Objekt-Aufnahmeschienen.

Vorteilhafterweise weisen die Befestigungsnippel einen annähernd kreisförmigen Querschnitt und zwischen ihrem äußeren Ende und der Objekt-Aufnahmeschiene einen gegenüber dem äußeren Ende verringerten Querschnitt auf, wobei die Befestigungsösen in ihrem oberen Teil eine zur Aufnahme des äußeren Endes eines Befestigungsnippels und in ihrem unteren Teil eine zur Aufnahme des verringerten Querschnitts des Befestigungsnippels jeweils geeignete Form aufweisen. Die Befestigungsnippel der Objekt-Aufnahmeschienen werden in diesem Fall durch das obere, größere Ende der Befestigungsösen hindurchgeführt und dann nach unten, in den Teil mit verringertem Querschnitt gedrückt, so dass die Objekt-Aufnahmeschienen sicher in den Seitenwänden gehaltert sind. Von Vorteil ist es dabei insbesondere, dass der untere Teil der zur horizontal-verschiebbaren Halterung eines Befestigungsnippels vorgesehenen Befestigungsösen einen größeren Querschnitt aufweist als der verringerte Querschnitt des Befestigungsnippels.

Die Erfindung bezieht sich weiterhin auf einen Klimaschrank mit mindestens einer erfindungsgemäßen Objekt-Lagerstation.

Vorteilhafte Ausgestaltungen der Erfindung sind in den Unteransprüchen angegeben.

Nachfolgend wird ein Ausführungsbeispiel der Erfindung anhand einer Zeichnung näher erläutert.

### In der Zeichnung zeigt:

- Fig. 1: die schematische Darstellung eines Klimaschrankes mit Objekt-Lagerstation,
- Fig. 2: eine Objekt-Lagerstation in der Vorderansicht (Fig. 2 A),
in der Seitenansicht (Fig. 2 B),
in der Draufsicht (Fig. 2 C),
sowie in Detailausschnitten X (Fig. 2 D) und Y (Fig. 2 E),
- Fig. 3: eine Objekt-Aufnahmeschiene in Vorder- und Seitenansicht, sowie
- Fig. 4: eine Detailansicht einer Seitenwand der Objekt-Lagerstation mit ausschnittsweiser Vergrößerung einer Befestigungsöse.

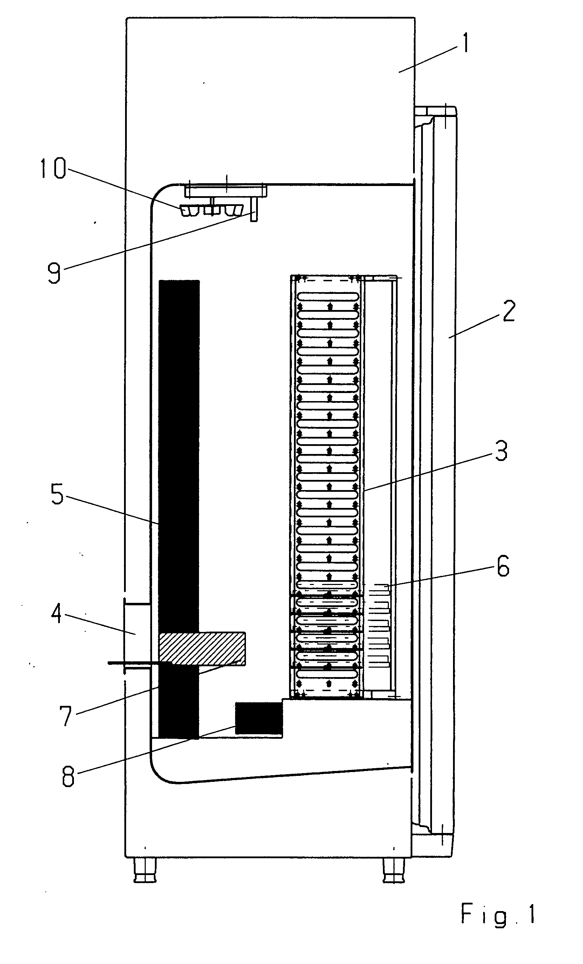

Figur 1 zeigt eine Seitenansicht, teilweise geschnitten, eines erfindungsgemäßen Klimaschrankes 1. Der Klimaschrank 1 weist eine Servicetür 2 auf, durch die die Objektlagerstationen 3 montiert und entnommen werden können und durch die Wartungsarbeiten möglich sind. Gegenüberlegend weist der Klimaschrank 1 eine Beschickungsöffnung 4 auf, durch die mittels eines Handlingsystems 5 Mikrotiterplatten 6 in den Klimaschrank 1 eingebracht und aus diesem entnommen werden können. Das Handlingsystem 5 besteht aus einem Lift zur senkrechten Positionierung von Mikrotiterplatten 6 und einer horizontal Verschiebeeinheit 7 zur horizontalen Positionierung bzw. Bewegung der Mikrotiterplatten 6. Derartige Anordnungen sind aus dem Stand der Technik, beispielsweise aus WO 98/05753 prinzipiell bekannt.

In der Objektlagerstation 3 ist eine Vielzahl von Mikrotiterplatten 6 übereinander angeordnet. Die Objektlagerstation 3 selbst ist auf einem Trägergestell 8 gehaltert. Im oberen Bereich des Klimaschrankes 1 sind eine Messzelle 9 und ein Ventilator 10 angeordnet.

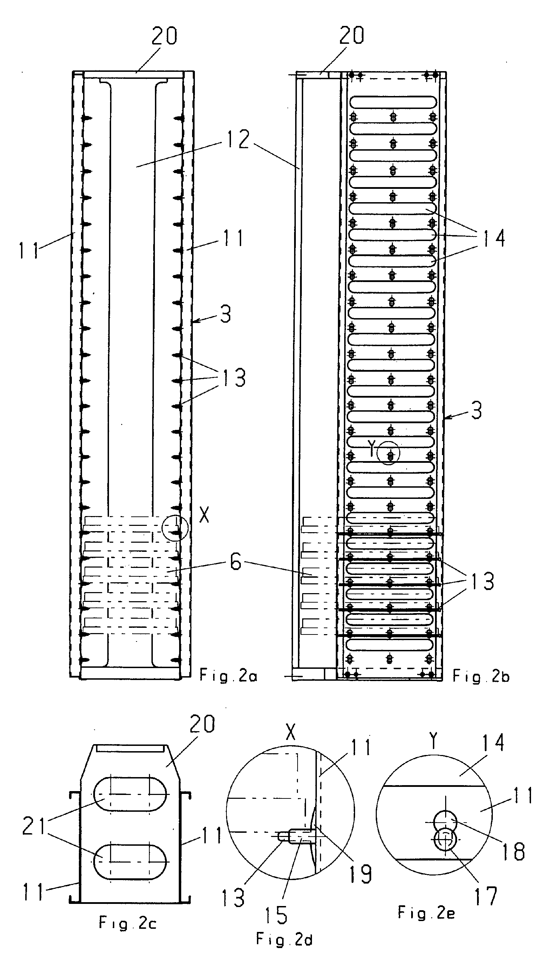

Details einer Objektlagerstation 3 sind in Fig. 2 dargestellt. Fig. 2 A zeigt die Vorderansicht einer Objektlagerstation 3 mit sich gegenüberliegenden Seitenwänden 11 und einer Rückwand 12, zwischen denen Mikrotiterplatten 6 übereinander angeordnet sind (in Fig. 2 lediglich andeutungsweise dargestellt). Die Mikrotiterplatten 6 sind auf Objekt-Aufnahmeschienen 13 gelagert. Zwischen den Objekt-Aufnahmeschienen 13 sind in den Seitenwänden 11 Durchbrüche 14 übereinander angeordnet (Fig. 2 B). Durchbrüche 14 und Objekt-Aufnahmeschienen 13 sind so angeordnet, dass die Oberkanten der Mikrotiterplatten 6 im Bereich der Durchbrüche 14 zwischen deren Ober- und Unterkante angeordnet sind. Die Durchbrüche 14 erstrecken sich in waagerechter Richtung. Dabei sind die Objekt-Aufnahmeschienen 13 jeweils im gleichen Abstand zu der Unterkante der Durchbrüche 14 angeordnet. An ihren Enden weisen die Objekt-Aufnahmeschienen 13 jeweils nach oben und unten über die Auflagefläche der Auflageschienen 13 hinausragende Anschlagelemente 15 (Fig. 2 D) auf, die ein Entgleiten der Mikrotiterplatten 6 aus der Objektlagerstation 3 heraus verhindern. Durch die symmetrische Anordnung der Anschlagelemente 15 ist ein wechselseitiges Einsetzen der Objekt-Aufnahmeschienen 13 an beiden Seiten der Objektlagerstation 3 möglich. Dadurch ist nur eine einzige Ausführung von Objekt-Aufnahmeschienen 13 notwendig.

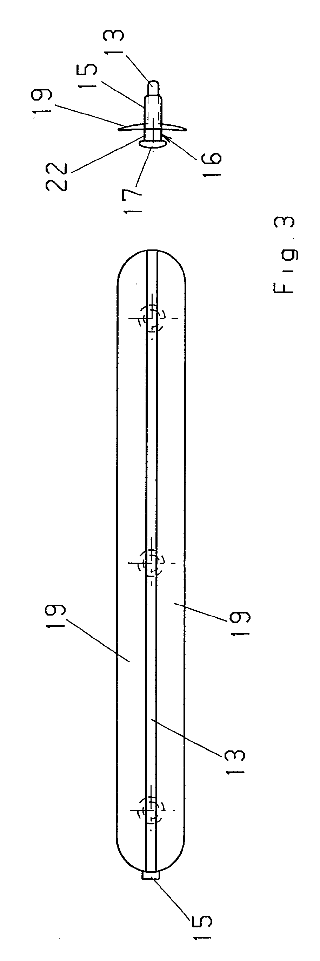

In den Fig. 2 D und 2 E ist die Befestigung der Objekt-Aufnahmeschienen 13 in der Seitenwand 11 dargestellt. Die Objekt-Aufnahmeschienen 13 weisen zur Befestigung an den Seitenwänden 11 Befestigungsnippel 16 (Fig. 3) auf, deren äußeres Ende 17 an der nach außen weisenden Oberfläche der Seitenwand 11 anliegt. Die Befestigungsnippel 16 sind in Befestigungsösen 18 gehaltert. Auf der Innenseite der Seitenwand 11 ist die Objekt-Aufnahmeschiene 13 mittels Stützleisten 19 abgestützt, wobei sich die Stützleisten 19 oberhalb und unterhalb der Objekt-Aufnahmeschiene 13 an der Seitenwand 11 anliegend erstrecken. Ihr Querschnitt vergrößert sich stetig vom äußeren Ende der Stützleisten 19 zu der Verbindungsstelle mit der Objekt-Aufnahmeschiene 13 hin, er bildet eine Art schiefe Ebene, auf der eine oberhalb der Objekt-Aufnahmeschiene 13 eingesetzte Mikrotiterplatte 6 nach unten bis auf die Objekt-Aufnahmeschiene 13 gleitet und dadurch zentriert wird. Im Detail ist eine Objekt-Aufnahmeschiene 13 mit darin angeordneter Stützleiste 19 in Fig. 3 sowohl in der Frontals auch in der Seitenansicht dargestellt. Die Stützleisten 19 können zu ihren Außenkanten hin leicht gebogen sein, so dass sie mit Oberkante und Unterkante unter Spannung an den Seitenteilen anliegen. Durch die Federwirkung der Stützleisten ist ein fester, nichtwackelnder Sitz gewährleistet.

Fig. 2 C zeigt die Draufsicht auf eine Objekt-Lagerstation 3. Die Deckfläche 20 weist Grifföffnungen 21 auf, die dem Transport der Objekt-Lagerstation 3 dienen. Die Seitenwände 11 sind an ihrem vorderen und hinteren Ende mehrfach abgewinkelt, um bei möglichst geringer Materialstärke eine hohe Festigkeit zu erzielen.

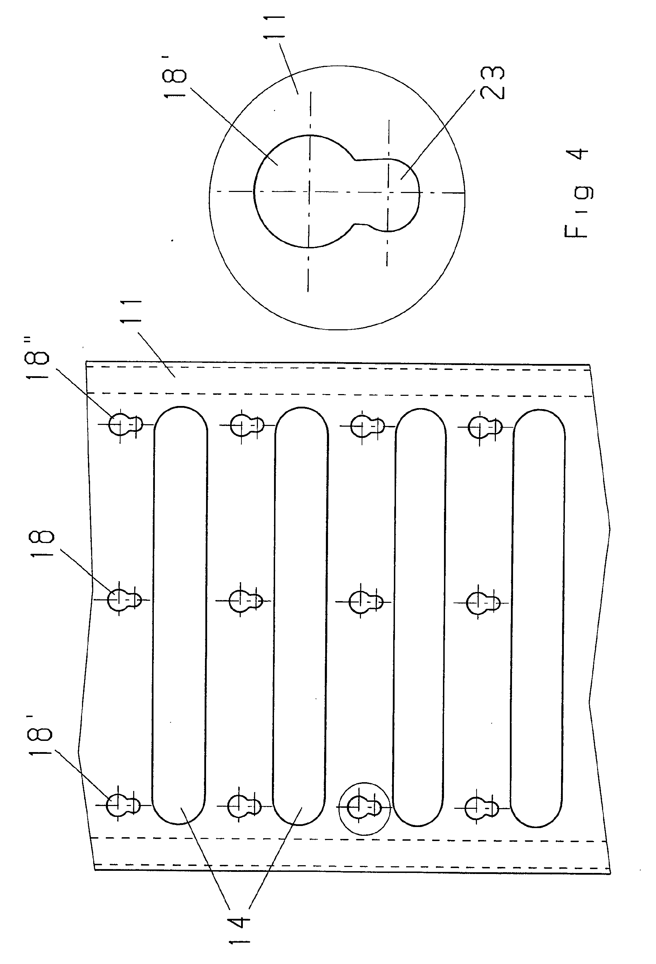

Fig. 4 zeigt einen Ausschnitt aus einer Seitenwand 11 mit Einzelheiten der zwischen den Durchbrüchen 14 angeordneten Befestigungsösen 18; 18'; 18". Die Befestigungsösen 18 sind mittig zwischen den beiden Befestigungsösen 18' und 18" angeordnet und symmetrisch ausgebildet. Sie weisen die Form eines traditionellen Schlüssellochs mit einem oberen, weiteren Teil und einem unteren, engeren Teil auf, wobei der obere kreisförmige Teil einen größeren Querschnitt aufweist als das äußere Ende 17 des zugehörigen Befestigungsnippels. Dadurch kann dieser durch die Befestigungsöse 18 hindurchgeführt werden. Die Objekt-Aufnahmeschiene 13 wird anschließend abgesenkt, so dass der Teil des Befestigungsnippels 16 mit dem geringeren Querschnitt 22 in dem unteren, kleineren Teil der schlüssellochförmigen Befestigungsöse 18 angeordnet wird. Der untere Teil ist in seiner Größe der Größe des verringerten Querschnitts 22 des Befestigungsnippels 16 angepaßt, so dass dieser in der Befestigungsöse 18 arretiert ist. Um ein unbeabsichtigtes Heben der Objekt-Aufnahmeschiene 13 zu verhindern, kann die Übergangsstelle zwischen dem oberen und dem unteren Teil des Schlüssellochs einen solchen Querschnitt aufweisen, der geringfügig kleiner ist als der verringerte Querschnitt 22 der Befestigungsöse 16, so dass diese mit Druck und aufgrund ihrer Elastizität bzw. der Elastizität des Materials der Seitenwand 11 durch diese verengte Stelle hindurch gepresst wird.

Die Befestigungsösen 18' und 18" sind prinzipiell gleich ausgebildet wie die mittlere Befestigungsöse 18. Im Unterschied zu der Befestigungsöse 18 weisen die äußeren Befestigungsösen 18', 18" jedoch in ihrem unteren, engeren Teil in waagerechter Richtung einen größeren Querschnitt auf als der verringerte Querschnitt 22 des Befestigungsnippels 16, so dass dieser innerhalb des unteren Teils 23 der Befestigungsösen 18'; 18" horizontal beweglich ist. Die Vergrößerung ist im gezeigten Beispiel (Fig. 4) asymmetrisch ausgebildet und jeweils nach außen von der mittleren Befestigungsöse 18 wegweisend ausgebildet, so dass eine sich bei Temperaturerhöhung mehr als die Seitenwand 11 ausdehnende Objekt-Aufnahmeschiene 13 in Längsrichtung frei beweglich ist und nicht unter Spannung gesetzt wird.

Natürlich ist es möglich, Befestigungsösen oder Befestigungsnippel in anderer geeigneter Form auszuführen.

## Patentansprüche

1. Objektlagerstation mit einer Vorderfront zur Beschickung mit Objekten, einer Rückseite und zwei gegenüberliegend angeordneten Seitenwänden und mit an den Seitenwänden jeweils paarweise gegenüberliegend im Inneren der Objektlagerstation im Wesentlichen horizontal erstreckend angeordneten Objekt-Aufnahmeschienen mit Auflageflächen,
**dadurch gekennzeichnet,**
**dass** die Seitenwände (11) mehrere gleiche, sich waagerecht erstreckende, übereinander angeordnete Durchbrüche (14) aufweisen, die in beiden Seitenwänden (11) in gleicher Anzahl und Lage und in stets gleichem Abstand von benachbarten Durchbrüchen (14) angeordnet sind, wobei die Anzahl der Durchbrüche (14) jeder Seitenwand (11) der Anzahl der Objekt-Aufnahmeschienen (13) der Seitenwand (11) entspricht und dass jeder Durchbruch (14) jeweils den gleichen Abstand zu der jeweils nächstliegenden Objekt-Aufnahmeschiene (13) aufweist.

2. Objektlagerstation nach Anspruch 1 oder 2,
**dadurch gekennzeichnet,**
**dass** die Objekt-Aufnahmeschienen (13) unterhalb der Durchbrüche (14) angeordnet sind.

3. Objektlagerstation nach Anspruch 1 oder 2,
**dadurch gekennzeichnet,**
**dass** die Objekt-Aufnahmeschienen (13) lösbar an den Seitenteilen (11) angeordnet sind.

4. Objektlagerstation nach einem der Ansprüche 1 bis 3,
**dadurch gekennzeichnet,**
**dass** die Objekt-Aufnahmeschienen (13) zumindest an ihrem der Vorderfront zugewandten Ende auf ihrer Oberseite jeweils mindestens ein Anschlagelement (15) aufweisen.

5. Objektlagerstation nach Anspruch 4,
**dadurch gekennzeichnet,**
**dass** an den Ober- und Unterseiten der Objekt-Aufnahmeschienen (13) an deren der Vorderfront und/oder der Rückseite zugewandten Enden jeweils gleiche Anschlagelemente (15) angeordnet sind.

6. Objektlagerstation nach einem der Ansprüche 1 bis 5,
**dadurch gekennzeichnet,**
**dass** an den den Seitenwänden (11) zugewandten Kanten der Objekt-Aufnahmeschienen (13) Stützleisten (19) angeordnet sind, die an der jeweiligen Seitenwand (11) anliegen und sich mindestens oberhalb der Auflagefläche der Objekt-Aufnahmeschienen (13) erstrecken, wobei der senkrecht zur Seitenwand (11 ) und senkrecht zur Auflagefläche der Objekt-Aufnahmeschiene (13) gebildete Querschnitt der Stützleisten (19) an der Verbindungsstelle zur Objekt-Aufnahmeschiene (13) eine größere, senkrecht zur Seitenwand (11) gemessene Breite aufweist als an dem der Verbindungsstelle abgewandten Ende.

7. Objektlagerstation nach Anspruch 6,
**dadurch gekennzeichnet,**
**dass** die Breite des Querschnitts der Stützleisten (19) stetig geändert ist.

8. Objektlagerstation mit einer Vorderfront zur Beschickung mit Objekten, einer Rückseite und zwei gegenüberliegend angeordneten Seitenwänden und mit an den Seitenwänden jeweils paarweise gegenüberliegend im Inneren der Objektlagerstation im Wesentlichen horizontal erstreckend angeordneten Objekt-Aufnahmeschienen mit Auflageflächen,
**dadurch gekennzeichnet,**
**dass** die Objekt-Aufnahmeschienen (13) jeweils in mindestens zwei in den Seitenwänden (11) horizontal nebeneinander angeordneten Befestigungsösen (18;18';18") mittels an den Aufnahmeschienen (13) angeordneter Befestigungsnippel (16) gehaltert sind, wobei die Befestigungsösen (18;18';18") sich in vertikaler Richtung erstrecken und wobei ein Befestigungsnippel (16) jeweils einer Objekt-Aufnahmeschiene (13) im unteren Bereich der zugeordneten Befestigungsöse (18) arretiert ist und der mindestens eine weitere Befestigungsnippel (16) im unteren Bereich der zugeordneten Befestigungsöse (18';18") horizontal-verschiebbar gehalten ist.

9. Objektlagerstation nach Anspruch 8,
**dadurch gekennzeichnet,**
**dass** die Objekt-Aufnahmeschienen (13) jeweils mit drei Befestigungsnippeln (16) in einer Seitenwand (11) gehaltert sind.

10. Objektlagerstation nach Anspruch 9,
**dadurch gekennzeichnet,**
**dass** der mittlere Befestigungsnippel (16) in der zugeordneten Befestigungsöse (18) arretiert ist.

11. Objektlagerstation nach einem der Ansprüche 7 bis 10,
**dadurch gekennzeichnet,**
**dass** die Befestigungsnippel (16) einen annähernd kreisförmigen Querschnitt und zwischen ihrem äußeren Ende (17) und der Objekt-Aufnahmeschiene (13) einen gegenüber dem äußeren Ende (17) verringerten Querschnitt (22) aufweisen und wobei die Befestigungsösen (18;18';18") in ihrem oberen Teil eine zur Aufnahme des äußeren Endes (17) der Befestigungsnippel (16) und in ihrem unteren Teil eine zur Aufnahme des verringerten Querschnitts (22) der Befestigungsnippel (16) jeweils geeignete Form aufweisen.

12. Objektlagerstation nach Anspruch 11,
**dadurch gekennzeichnet,**
**dass** der untere Teil der zur horizontal-verschiebbaren Halterung eines Befestigungsnippels (16) vorgesehenen Befestigungssösen (18;18';18") einen größeren Querschnitt aufweist als der verringerte Querschnitt (22) des Befestigungsnippels (16).

13. Objektlagerstation nach einem der Ansprüche 8 bis 12,
**dadurch gekennzeichnet,**
**dass** die Objekt-Aufnahmeschienen (13) zumindest an ihrem der Vorderfront zugewandten Ende auf ihrer Oberseite jeweils mindestens ein Anschlagelement (15) aufweisen.

14. Objektlagerstation nach Anspruch 13,
**dadurch gekennzeichnet,**
**dass** an den Ober- und Unterseiten der Objekt-Aufnahmeschienen (13) an deren der Vorderfront und der Rückseite zugewandten Enden jeweils gleiche Anschlagelemente (15) angeordnet sind.

15. Objektlagerstation nach einem der Ansprüche 8 bis 14,
**dadurch gekennzeichnet,**
**dass** an den den Seitenwänden (11) zugewandten Kanten der Objekt-Aufnahmeschienen (13) Stützleisten (19) angeordnet sind, die an der jeweiligen Seitenwand (11) anliegen und sich mindestens oberhalb der Auflagefläche der Objekt-Aufnahmeschienen (13) erstrecken, wobei der senkrecht zur Seitenwand (11 ) und senkrecht zur Auflagefläche der Objekt-Aufnahmeschiene (13) gebildete Querschnitt der Stützleisten (19) an der Verbindungsstelle zur Objekt-Aufnahmeschiene (13) eine größere, senkrecht zur Seitenwand (11) gemessene Breite aufweist als an dem der Verbindungsstelle abgewandten Ende.

16. Objektlagerstation nach Anspruch 15,
**dadurch gekennzeichnet,**
**dass** die Breite des Querschnitts stetig geändert ist.

17. Klimaschrank,
**dadurch gekennzeichnet,**
**dass** er mindestens eine Objektlagerstation (3) nach mindestens einem der Ansprüche 1 bis 16 aufweist.
